Europäisches Patentamt

European Patent Office

Office européen des brevets

(19)

(11) Publication number : **0 446 069 B1**

# (12) EUROPEAN PATENT SPECIFICATION

(45) Date of publication of patent specification :
**01.09.93 Bulletin 93/35**

(51) Int. Cl.⁵ : **A61K 35/74, // (A61K35/74, 31:70)**

(21) Application number : **91301955.0**

(22) Date of filing : **08.03.91**

(54) **Treatment of Clostridium difficile diarrhoea and pseudomembranous colitis.**

(30) Priority : **09.03.90 JP 56596/90**

(43) Date of publication of application :
**11.09.91 Bulletin 91/37**

(45) Publication of the grant of the patent :
**01.09.93 Bulletin 93/35**

(84) Designated Contracting States :
**CH DE FR GB IT LI**

(56) References cited :
**FR-M- 2 678
GB-A- 1 190 386
CHEMICAL ABSTRACTS, vol. 107, 20 July 87
Columbus, Ohio, USA TAKASHI, KATSUO:
"Studies on the anti-diarrheal activity of Clostridium butyricum Miyairi 588" page 55; column 2; ref. no. 17606**

(73) Proprietor : **KABUSHIKI KAISHA MIYARISAN
SEIBUTSU IGAKU KENKYUSHO
10-3 Kaminakazato 1-chome, Kita-ku
Tokyo (JP)**

(72) Inventor : **Kuroiwa, Toyoaki
2084 Oaza Togura, Togura-machi
Hanishina-gun, Nagano-ken (JP)**
Inventor : **Taguchi, Nobuhiro
Nipposo, 9981, Ichinomiya, Ichinomiya-machi
Chosei-gun, Chiba-ken (JP)**

(74) Representative : **Sheard, Andrew Gregory et al
Kilburn & Strode 30, John Street
London WC1N 2DD (GB)**

EP 0 446 069 B1

## Description

This invention relates to preventive and/or curative pharmaceutical compositions for *Clostridium difficile* diarrhoea and pseudomembranous colitis.

As soon as a child is born, certain species of microorganisms begin to inhabit his intestinal tract and eventually form a substantially stable intestinal microflora after their population reaches equilibrium. Though the individual microorganisms of which the intestinal microflora is composed may do either harm or good to the host, depending on their abilities to produce or decompose substances, it has been pointed out that the intestinal microflora as a whole has some bearing on supply of vitamins, prevention of infection, and intestinal functions (peristalsis and absorption). The state of a host's intestinal microflora profoundly concerns the health of the host.

The intestinal microflora varies according to various factors such as the host's physiological condition, foods, medicines and stress. Particularly in recent years, numerous kinds of antibacterial agents possessing outstanding antibacterial power and a wide antibacterial spectrum have been offered in large quantities to the field of medicine and have been finding utility for the purpose of curing and preventing various infectious diseases. Incidentally, it is known that administration of an antibacterial agent itself constitutes the greatest factor affecting the intestinal microflora and, by disrupting the host's normal intestinal microflora, induces abnormal propagation of a pathogen otherwise curbed under the normal conditions and consequently can cause outbreak of diarrhoea, colitis, etc. Particularly, in the clinic, the *Clostridium difficile* diarrhoea and pseudomembranous colitis due to the toxin yielded by *Clostridium difficile*, which is abnormally propagated intestinally in a patient subjected to administration of an antibacterial agent, have been attracting attention as serious and possibly fatal diseases.

Treatment of *Clostridium difficile* diarrhoea and pseudomembranous colitis has had to involve discontinuation of the antibacterial agent, a change in the kind of antibacterial agent being used, and then administration of an intestinal bactericide which is highly active against *Clostridium difficile*, a microorganism responsible for the diseases. No easy elimination of the diseases, however, is attained even when the chemotherapy is continued for an extended period for a number of reasons: the therapy given for a previous infectious disease continue to have its effect; the host's intestinal microflora is further disturbed by the antibacterial property of the intestinal bactericide itself; the disturbed microflora is susceptible to the occurrence of a new infection; the pathogen has the possibility of acquiring antibacterial resistance; and the *Clostridium difficile* in the intestines forms spores inherently resistant to antibacterial agents. When the therapy is discontinued, the spores of *Clostridium difficile* which have survived germinate and propagate to cause a recurrence of the diseases and seriously enervate the patient. In fact, the chemotherapy demands advanced clinical judgement supported by much experience. An effective preventive and curative method for these diseases is needed.

From FR-M-2678 a pharmaceutical composition comprising cells or spores of a butyric acid bacterium and a saccharide is known.

A problem underlying this invention is to provide an improved preventive and curative medical composition for *Clostridium difficile* diarrhoea and pseudomembranous colitis.

This problem is solved by providing a novel preventive and/or curative pharmaceutical composition for *Clostridium difficile* diarrhoea and pseudomembranous colitis, which composition comprises cells or spores of a butyric acid bacterium and a polysaccharide. The term "pharmaceutical composition" as used herein includes a veterinary composition.

The present invention, as described above, is directed to a preventive and/or curative pharmaceutical composition for *Clostridium difficile* diarrhoea and pseudomembranous colitis, which medical composition comprises cells or spores of a butyric acid bacterium and a polysaccharide. The administration of such a pharmaceutical composition prevents and/or cures the diseases mentioned above and, at the same time, is advantageous in that the toxicity of the composition is low.

Preferred embodiments of the invention will now be described.

There has been a report of the effective use of a butyric acid bacterium against *Clostridium difficile* diarrhoea in morbidity models in small experimental animals, namely golden hamsters. The present inventors conceived an idea for developing preventative and curative pharmaceuticals for *Clostridium difficile* diarrhoea and pseudomembranous colitis in human beings, based on their knowledge that: (1) butyric acid bacteria resist putrefaction; (2) they approximate *Clostridium difficile* in their biochemical nature so much so that they would be likely to compete with *Clostridium difficile* in the intestinal tract; and (3) they do not affect the host's normal microflora. This idea, however, turned out to be impracticable because the amount of butyric acid bacterium that would have to be administered to a human being as indicated in the report is extremely large (exceeding 1 kg/day per adult); this dose surpasses the acute oral toxicity of a butyric acid bacterium whose $LD_{50}$ is 5,000 mg/kg, as determined in an experimental system using rats); further doubts exist as to the efficacy of the butyric acid bacterium in the light of animals preference for the taste of ground dry bacterial cells administered and difference of intestinal microflora between animal species.

The present inventors have made a comparative study on the intestinal microflora between hamsters and human beings. They have found that the micro-organic density in human beings is about 10 times higher than that in hamsters and, further, that for the action of the butyric acid bacterium to be manifested as expected, the intestinal concentration of the butyric acid bacterium should be proportionately increased. As one measure, they prepared a powdered formulation by kneading a paste of *Clostridium butyricum* MIYAIRI 588 (hereinafter referred to as "CbM"), a species of butyric acid bacterium, with corn starch, and subsequently drying the resulting blend. Corn starch is a saccharide readily usable as a carbon source by the strain and incapable of being directly absorbed through the intestinal wall. The mixing ratio of CbM and corn starch in this formulation was fixed. "Miya BM" (a blend of 40mg of ground CbM cells with 960mg of corn starch and containing $10^8$ CFU of CbM spores per gram) was administered, as a powdered CbM formulation, to a human patient to whom was administered an antibacterial agent; the patient's faeces were examined to find the recovery ratio. In this experiment, even at a small dose ($10^7$ CFU/kg) the formulation showed a high recovery ratio ($10^7$ CFU/g of faeces) as compared with that found in the experiment using hamsters; this suggests a successful increase in intestinal CbM concentration. The powdered pharmaceutical was virtually free from the odour peculiar to CbM, and the corn starch incorporated in it helped enhance its taste. Then, hospital in-patients who had no particularly notable disease of the digestive tract and were undergoing therapy with an antibacterial agent were caused to submit their faeces prior to administration of the antibacterial agent. These in-patients were subsequently divided into a first group, subjected to administration of one to three doses each of 1g of the powdered CbM pharmaceutical per day, and a second group, not subjected to the administration; the two groups of in-patients submitted their faeces similarly. Examination of their faeces revealed that, in the CbM administration group, in-patients in whose faeces *Clostridium difficile* or its toxin was detected accounted for 5% of the total, compared to 30% found in the group of in-patients not subjected to CbM administration. This indicates that CbM administration was conspicuously effective in curbing the intestinal propagation of *Clostridium difficile* in humans. When in-patients who complained of abdominal disorders after administration of an antibacterial agent and in whose faeces *Clostridium difficile* was detected were treated with one to three doses each of 1g of "Miya BM" per day, as a powdered CbM medicine, their abdominal conditions and faecal conditions improved. On the 10th day of administration of "Miya BM", *Clostridium difficile* was not found in their faeces. This is the basis of the present invention. CbM is an anaerobic sporal bacterium which propagates and forms spores in an ordinary culture medium under anaerobic conditions. CbM forms spores in an ordinary culture medium under anaerobic conditions. Bouillon, incorporating 2% corn starch, for example, serves as a suitable culture medium therefor.

Polysaccharides which are usable in the present invention include polysaccharides which can be assimilated by butyric acid bacteria. Polysaccharides include, for example, starch, dextrin, glycogen, pectin, raffinose, amygdalin and glycomannan. Raffinose and starch prove to be particularly desirable. Such a polysaccharide may be incorporated in compositions of the present invention in an amount in the range of 1mg to 10g, preferably 10mg to 10g, per $10^8$ CFU of the cells or spores of the butyric acid bacterium.

A pharmaceutical composition of the present invention may be administered in an amount in the range of from $10^6$ to $10^9$ CFU, preferably from $10^6$ to $10^8$ CFU, as cells or spores of the butyric acid bacterium per kg of human body weight. The composition can be orally administered to human beings or animals, combined, when necessary, or desirable, with a pharmaceutically acceptable carrier.

The term "butyric acid bacteria" as used herein refers to microorganisms which yield butyric acid as a product of metabolism. Among other butyric acid bacteria, *Clostridium butyricum* proves to be particularly desirable.

Antibacterial agents whose administration may lead to the proliferation of *Clostridium difficile* contemplated herein include penicillins, cephalosporins, aminoglycosides, macrolides, lincosamides, tetracyclines, peptides, depsipeptides, rifamycins, sulphonamides, pyridon carboxylic acids, nitrofurans, nitroimidazoles, antituberculosis agents, antineoplastic agents, antimycotics, trimethoprim, chloramphenicol, novobiocin, fusidic acid, fosfomycin and cycloserine. Pharmaceutical compositions of the present invention exhibit extremely low acute and chronic toxicity.

The present invention will now be illustrated more specifically below with reference to working examples.

## EXAMPLES

*Clostridium butyricum* MIYAIRI 588 used in the following experiments has been deposited at the Fermentation Research Institute under FERM-BP No. 2789.

Example 1

Faeces from 15 golden hamsters (70g, male) and faeces from 69 patients who had no particularly notable disease in their digestive tracts and had not yet been treated with an antibacterial agent had their intestinal microflora determined by methods known in the art. It can clearly be seen from the results given in Table 1 that, in human intestinal microflora, anaerobic microorganisms were predominant and that the micro-organic concentration per gram of faeces was 10 times as high as that in the faeces of the hamsters.

Example 2

A suspension of 5.25mg potency of cefatrizine (hereinafter referred to briefly as "CFT"), in purified water was orally administered once, with the aid of a catheter, to five golden hamsters (70g, male). Then a suspension in purified water of 1ml of ground dry cells of CbM, containing $10^9$ CFU of CbM spores, was orally administered to each of the hamsters, divided in three doses daily. Faeces were taken from the recta of the hamsters on the fifth day of administration. To five patients who had no particularly notable disease in their digestive tracts, and who were undergoing therapy using antibacterial agents (penicillins and cephalosporins) one to three doses each of 1g of "Miya BM" (a blend of 40mg of ground CbM cells with 960mg of corn starch and containing $10^8$ CFU of CbM spores per gram) were administered daily. Faeces were taken from the five patients. These faeces were examined to determine counts of live CbM bacteria. The culture (37°C, 72 hrs) for isolation of CbM was carried out anaerobically in 5% horse blood BL Agar containing 350mg of cycloserine, 1mg of kanamycin, 75μg of clindamycin, and 12mg of trimethoprim each per litre of the culture medium. As shown in Table 2, CbM was detected in the samples from all ten subjects. The recovered CbM concentrations in the samples from the patients administered "Miya BM" in the form of a powdered CbM pharmaceutical were higher than those in the samples from the hamsters administered ground CbM cells. The efficacy of administration, in terms of dose per body weight, observed in the administration to human beings of "Miya BM" as a powdered CbM pharmaceutical, was 1,000 times higher than that observed in the case of hamsters. This implies that the introduction of the CbM spores as deposited, fast on corn starch, to the intestinal interior resulted in the promotion of the propagation of CbM for certain reasons, such as the fact that corn starch is an excellent carbon source for CbM. The microflora in faeces reflects the intestinal microflora. For the CbM to manifest its action in the human intestinal tract as expected, it is particularly desirable that a CbM pharmaceutical composition, combining CbM with corn starch, should be used as an oral medicine.

Example 3

In-patients who had no particularly notable disease in their digestive tracts, and who were to be treated using antibacterial agents, were caused to offer their faeces as samples before the administration of the antibacterial agents. After the start of the antibacterial therapy (using penicillins and cephalosporins), in-patients were divided into a first group (20), subjected to the administration of one to three doses each of 1g of "Miya BM" (a blend of 40mg of ground CbM cells with 960mg of corn starch and containing $10^8$ CFU of CbM spores per gram) as a powdered CbM medicine daily, and a second group (20) who were not subjected to the administration. Faeces were taken from these two groups of in-patients. The faeces were examined for detection of *Clostridium difficile* and its toxin. Detection of *Clostridium difficile* was carried out by anaerobically culturing a sample in a CCFA culture medium (37°C, 72 hrs; with the detection limit of culture at 400 CFU/g of faeces) and toxin detection was carried out by the use of a latex reagent [detection of the D-1 toxin of *Clostridium difficile* possible above 500ng/ml (sample)]. The results were as shown in Table 3. It can clearly be seen from the results that, in the CbM administration group, the patients in whose faeces *Clostridium difficile* or its toxin was detected accounted for 5% of all the patients of the CbM administration group, as compared with 30% found in the patients of the group not administered CbM. This implies that the administration of CbM was particularly effective in curbing the propagation of *Clostridium difficile* in the human intestinal tract. The clinically isolated strain of *Clostridium difficile* was found to possess the behaviour shown in Table 4.

Example 4

In in-patients who had no particularly notable disease in their digestive tracts, and who were undergoing therapy using an antibacterial agent (oral grade ampicillin), the two in-patients in whose faeces *Clostridium difficile* was detected were given one to three doses each of 1g of "Miya BM" as a powdered CbM containing pharmaceutical daily. The results are shown in Table 5. It can clearly be seen from the results that the two in-patients both showed an improvement in their faecal state. Faeces taken on the 10th day of the administration of "Miya BM" showed no culture of *Clostridium difficile*, but CbM was detected. The abdominal disorders which these two in-patients initially complained of ceased on the second to third day of the administration of "Miya BM". The behaviour of the clinically isolated strain of *Clostridium difficile* was as shown in Table 6.

### Table 1
### Intestinal microfloras in human beings and hamsters

|  | Human beings (N = 69) | Hamsters (N = 15) |
|---|---|---|
|  | Log No. CFU/g of faeces (ratio of detection) | Log No. CFU/g of faeces (ratio of detection) |
| Enterobacteriaceae | 7.8 ± 1.6( 99) | 6.3 ± 0.8(100) |
| Enterococcus | 7.9 ± 1.2(100) | 6.5 ± 0.6(100) |
| Staphylococcus | 3.5 ± 0.9( 65) | 4.0 ± 0.8(100) |
| Yeasts | 4.4 ± 1.3( 83) | 3.8 ± 1.0( 40) |
| Lactobacillus | 5.9 ± 2.1( 94) | 8.7 ± 0.6(100) |
| Bifidobacterium | 9.5 ± 0.8( 86) | 8.9 ± 0.5(100) |
| Eubacterium | 9.8 ± 1.3( 99) | 9.0 ± 0.7(100) |
| Bacteroidaceae | 10.2 ± 1.4( 99) | 8.4 ± 1.0(100) |
| Veillonella | 6.6 ± 1.7( 68) | 4.9 ± 1.1( 67) |
| Peptococcaceae | 9.3 ± 0.6( 86) | 8.5 ± 0.7( 93) |
| Clostridium | 9.9 ± 0.6(100) | 4.3 ± 1.0( 80) |
| C.perfringens | 5.2 ± 1.4( 48) | 0      (0) |
| Facultative Anaerobes | 8.6 ± 0.8(100) | 8.7 ± 0.5(100) |
| Obligate Anaerobes | 10.7 ± 0.5(100) | 9.6 ± 0.4(100) |
| Total Counts | 10.8 ± 0.4(100) | 9.6 ± 0.4(100) |

### Table 2
### Recovery of CbM from feces (N = 5)

|  | CbM administered, Log No. CFU/kg (body weight)/day | CbM recovered Log No. CFU/g (faeces) |
|---|---|---|
| Human beings | 7.0 | 6.7 ± 0.7 |
| Hamsters | 9.0 | 5.8 ± 1.0 |

Table 3
Effect of administration of CbM

| Clostridium difficile | Before administraion of antibacterial agent | After administration of antibacterial agent | |
|---|---|---|---|
| | Number of patients 40 | Number of patients not subjected to administration of Miya BM 20 | Number of patients subjected to administration of Miya BM 20 |
| Yielded (+), D-1toxin(-) | 0/40 | 1/20 | 0/20 |
| Yielded (-), D-1toxin(+) | 0/40 | 2/20 | 1/20 |
| Yielded (+), D-1toxin(+) | 0/40 | 3/20 | 0/20 |
| Total (ratio of detection) | 0/40 (0%) | 6/20 (30%) | 1/20 (5%) |

## Table 4
### Biochemical characteristics of clinically isolated strain
### (014-5, 053-3 and 055-2)

|  | 014-5 | 053-3 | 055-2 |
|---|---|---|---|
| Gram's stain | + | + | + |
| Formation of endospores | + | + | + |
| Aerobic growth | - | - | - |
| Motility | + | + | + |
| Haemolytic capacity | - | - | - |
| Milk | - | - | - |
| Gelatin | + | + | + |
| Nitrate | - | - | - |
| Indole | - | - | - |
| Esclin | + | + | + |
| Lecithinase | - | - | - |
| Lipase | - | - | - |
| Sugar-utilizing ability |  |  |  |
| Fructose | + | + | + |
| Glucose | + | + | + |
| Mannitol | + | + | + |
| Mannose | + | + | + |
| Xylose | - | - | - |
| Lactose | - | - | - |
| Maltose | - | - | - |
| Starch | - | - | - |
| Sucrose | - | - | - |
| Product of PYG |  |  |  |
| Acetic acid | + | + | + |
| Butyric acid | + | + | + |
| Iso-butyric acid | + | + | + |
| Valeric acid | + | + | + |
| Iso-valeric acid | + | + | + |
| Iso-caproic acid | + | + | + |
| Lactic acid | + | + | + |

7

Table 5
Effect of administration of CbM

| Clostridium difficile D-1 toxin in faeces[1] Number of bacteria in faeces[2] Log No. CFU/g of faeces | Before administration of Miya BM | | After administration of Miya BM | |
|---|---|---|---|---|
| | F.A. | T.S. | F.A. | T.S. |
| | -<br>6.5 | +<br>7.6 | -<br>max. 2.6 | -<br>max. 2.6 |
| CbM Number of live bacteria in faeces Log No. CFU/g of faeces | max. 2.6 | max. 2.6 | 7.3 | 7.6 |
| Faecal state | Large content of undigested food in faeces | Soft passage | Soft passage | Normal faeces |

1) Positive case of detection indicated by + and negative case of detection by-.
2) The number of live bacteria in faeces was measurable above 2.6 (Log No. CFU/g of faeces).

## Table 6
### Behaviour of clinically isolated strain
### (COOO1K-32 and COO2OK-25)

|  | C0001K-32 | C0020K-25 |
|---|---|---|
| Gram's stain | + | + |
| Formation of endospore | + | + |
| Aerobic growth | – | – |
| Motility | + | + |
| Haemolytic capacity | – | – |
| Milk | – | – |
| Gelatin | + | + |
| Nitrate | – | – |
| Indole | – | – |
| Esclin | + | + |
| Lecithinase | – | – |
| Lipase | – | – |
| Sugar-utilizing ability |  |  |
| Fructose | + | + |
| Glucose | + | + |
| Mannitol | + | + |
| Mannose | + | + |
| Xylose | – | – |
| Lactose | – | – |
| Maltose | – | – |
| Starch | – | – |
| Sucrose | – | – |
| Product of PYG |  |  |
| Acetic acid | + | + |
| Butyric acid | + | + |
| Iso-butyric acid | + | + |
| Valeric acid | + | + |
| Iso-valeric acid | + | + |
| Iso-caproic acid | + | + |
| Lactic acid | + | + |
| Fatal activity of hamsters [1] | + | + |

1) The sterilized filtrate of a culture broth (BHI) of an isolated strain was intra-abdominally administered in an amount of 1.0 ml to healthy hamsters (70 g, male).

## Claims

1. A pharmaceutical composition comprising cells or spores of a butyric acid bacterium and a polysaccharide.

2. A composition according to claim 1, wherein the butyric acid bacterium is *Clostridium butyricum*.

3. A composition according to claim 1 or 2, wherein the amount of the polysaccharide is in the range of 1mg to 10g, based on $10^8$ CFU of the cells or spores of the butyric acid bacterium

4. A composition according to any one of claims 1 to 3, wherein the polysaccharide is raffinose and/or starch.

5. The use of cells or spores of a butyric acid bacterium and a polysaccharide in the manufacture of an agent for the prophylaxis and/or treatment of *Clostridium difficile* diarrhoea and pseudomembranous colitis.

6. The use according to claim 5, wherein the butyric acid bacterium is *Clostridium butyricum*.

7. The use according to claim 5 or 6, wherein the amount of the polysaccharide is in the range of 1mg to 10g, based on $10^8$ CFU of the cells or spores of the butyric acid bacterium.

8. The use according to any one of claims 5 to 7, wherein the polysaccharide is raffinose and/or starch.

9. The use according to any one of claims 5 to 8, wherein the amount of cells or spores of the butyric acid bacterium is in the range of $10^6$ to $10^9$ CFU per kg human body weight.

10. A product comprising cells or spores of a butyric acid bacterium and a polysaccharide as a combined preparation for simultaneous, separate or sequential administration.


## Patentansprüche

1. Pharmazeutische Zusammensetzung, welche Zellen oder Sporen eines Buttersäurebakteriums und ein Polysaccharid enthält.

2. Zusammensetzung nach Anspruch 1, in welche das Buttersäurebakterium Clostridium butyricum ist.

3. Zusammensetzung nach den Ansprüchen 1 oder 2, in welchem der Gehalt des Polysaccharids im Bereich von 1 mg bis 10 g liegt, beruhend auf $10^8$ CFU (Kolonien bildenden Einheiten) an Zellen oder Sporen des Buttersäurebakteriums.

4. Zusammensetzung nach jedem der Ansprüche 1-3, in welcher das Polysaccharid Raffinose und/oder Stärke ist.

5. Verwendung von Zellen oder Sporen eines Buttersäurebakteriums und eines Polysaccharids bei der Herstellung einer Verbindung zur Prophylaxe und/oder Behandlung von Clostridium difficile-Diarrhoea und Pseudomembrancolitis.

6. Verwendung nach Anspruch 5, wobei das Buttersäurebakterium Clostridium butyricum ist.

7. Verwendung nach den Ansprüchen 5 oder 6, wobei der Gehalt der Polysaccharide im Bereich von 1 mg bis 10 liegt, beruhend auf $10^8$ CFU (Kolonien bildenden Einheiten) an Zellen oder Sporen des Buttersäurebakteriums.

8. Verwendung nach jedem der Ansprüche 5 bis 7, wobei das Polysaccharid Raffinose und/oder Stärke ist.

9. Verwendung nach jedem der Ansprüche 5-8, wobei der Gehalt an Zellen oder Sporen des Buttersäurebakteriums im Bereich von $10^6$ bis $10^9$ CFU (Kolonien bildenden Einheiten) pro kg menschliches Körpergewicht liegt.

10. Produkt mit Zellen oder Sporen eines Buttersäurebakteriums und einem Polysaccharid als Kombinationspräparat für gleichzeitige, getrennte oder aufeinanderfolgende Verabreichung.

**Revendications**

1. Une composition pharmaceutique comprenant des cellules ou des spores d'une bactérie productrice d'acide butyrique et un polysaccharide.

2. Une composition selon la revendication 1, dans laquelle la bactérie productrice d'acide butyrique est *Clostridium butyricum*.

3. Une composition selon la revendication 1 ou 2, dans laquelle la quantité du polysaccharide est dans la gamme de 1 mg à 10 g pour $10^8$ UFC des cellules ou des spores de la bactérie productrice d'acide butyrique.

4. Une composition selon l'une quelconque des revendications 1 à 3, dans laquelle le polysaccharide est le raffinose et/ou l'amidon.

5. L'utilisation de cellules ou de spores d'une bactérie productrice d'acide butyrique et d'un polysaccharide dans la fabrication d'un agent pour la prophylaxie et/ou le traitement de la diarrhée à *Clostridium difficile* et de la colite pseudo-membraneuse.

6. L'utilisation selon la revendication 5, dans laquelle la bactérie productrice d'acide butyrique est *Clostridium butyricum*.

7. L'utilisation selon la revendication 5 ou 6, dans laquelle la quantité du polysaccharide est dans la gamme de 1 mg à 10 g pour $10^8$ UFC de cellules ou de spores de la bactérie productrice d'acide butyrique.

8. L'utilisation selon l'une quelconque des revendications 5 à 7, dans laquelle le polysaccharide est le raffinose et/ou l'amidon.

9. L'utilisation selon l'une quelconque des revendications 5 à 8, dans laquelle la quantité des cellules ou des spores de la bactérie productrice d'acide butyrique est dans la gamme de $10^6$ à $10^9$ UFC par kg de poids corporel humain.

10. Un produit comprenant des cellules ou des spores d'une bactérie productrice d'acide butyrique et un polysaccharide, sous forme d'une préparation combinée pour l'administration simultanée, séparée ou successive.